(19) Europäisches Patentamt
European Patent Office
Office européen des brevets

(11) Veröffentlichungsnummer : **0 009 163**
**B1**

(12) # EUROPÄISCHE PATENTSCHRIFT

(45) Veröffentlichungstag der Patentschrift : **22.09.82**

(21) Anmeldenummer : **79103260.0**

(22) Anmeldetag : **04.09.79**

(51) Int. Cl.³ : **C 07 D235/20**, C 07 D407/04, C 07 D409/04, A 61 K 31/415// C07D333/38, C07D307/68, C07C123/00, C07D295/12

(54) **Substituierte Bisbenzimidazolylverbindungen, ihre Herstellung und Mittel gegen Protozoen- und Viruserkrankungen.**

(30) Priorität : **14.09.78 DE 2839989**

(43) Veröffentlichungstag der Anmeldung : **02.04.80 (Patentblatt 80/07)**

(45) Bekanntmachung des Hinweises auf die Patenterteilung : **22.09.82 Patentblatt 82/38**

(84) Benannte Vertragsstaaten : **AT BE CH DE FR GB IT NL**

(56) Entgegenhaltungen :
**DE A 2 711 362**
**NL A 268 089**
**US A 3 624 212**

(73) Patentinhaber : **HOECHST AKTIENGESELLSCHAFT**
**Postfach 80 03 20**
**D-6230 Frankfurt/Main 80 (DE)**

(72) Erfinder : **Rösner, Manfred, Dr.**
**Unter den Buchen 7**
**D-6239 Eppstein/Taunus (DE)**
Erfinder : **Raether, Wolfgang, Dr.**
**Falkensteinstrasse 6**
**D-6072 Dreieich (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 009 163 B1

Substituierte Bisbenzimidazolylverbindungen, ihre Herstellung und Mittel gegen Protozoen- und Viruserkrankungen

Gegenstand der Erfindung sind substituierte Bisbenzimidazolylverbindungen der allgemeinen Formel I

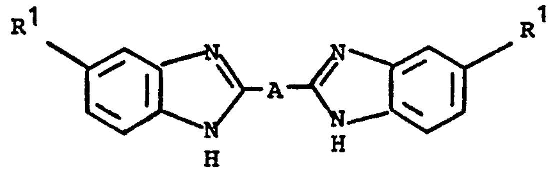

(I)

und deren physiologisch verträgliche Salze, worin A 2,5-Thiophendiyl, 2,5-Furandiyl, p-Phenylen, m-Phenylen oder 4,4'-Phenoxyphenylen bedeutet und worin $R^1$ durch eine der allgemeinen Formel IIa oder IIb wiedergegeben wird,

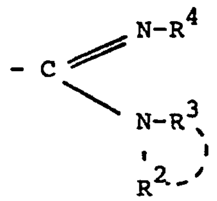

(IIa)

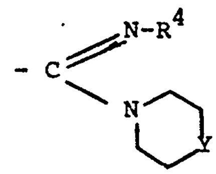

(IIb)

worin die Reste $R^2$ bis $R^4$ unabhängig voneinander Wasserstoff, Alkyl mit 1-18, vorzugsweise 1-12 C-Atomen wie Methyl, Äthyl, n—, iso-Propyl, n-, iso-, tert, -Butyl, n- oder iso-Pentyl, -Hexyl, -Heptyl, -Octyl, -Nonyl, -Decyl, -Undecyl oder Cycloalkyl mit 5-8 C-Atomen wie Cyclopentyl, Cyclohexyl, Cycloheptyl. Cyclooctyl, Aminoalkyl, N-Alkylaminoalkyl, N,N-Dialkylaminoalkyl, wobei eine Alkylgruppe jeweils 1-4 C-Atome aufweist, wie Aminomethyl, Aminoäthyl, N-Methylaminoäthyl, N,N-Dimethylaminoäthyl, N,N-Dimethylaminopropyl, N,N-Dibutylaminoäthyl, Morpholinoäthyl, Methoxyäthyl, Äthoxyäthyl, Benzyl, Phenäthyl oder Phenyl bedeuten, oder worin die Rest $R^2$ und $R^3$ zusammen eine Alkylenbrücke mit 4,5 oder 6 $CH_2$-Gruppen bedeuten, und worin Y O, S, NH oder N—$R^5$ bedeutet, wobei $R^5$ für Alkyl mit 1-4 C-Atomen steht.

Unter den Verbindungen der allgemeinen Formel I sind diejenigen bevorzugt, in denen A 2,5-Thiophendiyl, 2,5-Furandiyl, p-Phenylen oder 4,4'-Phenoxyphenylen bedeutet und die Substituenten $R^2$ bis $R^4$ der allgemeinen Formeln IIA oder IIb Wasserstoff, Alkyl mit 1-12 C-Atomen, Cycloalkyl mit 5-8 C-Atomen, Methoxyäthyl, Äthoxyäthyl, Phenylalkyl mit 1-2 C-Atomen im Alkylrest oder Phenyl bedeuten.

Besonders bevorzugt unter den Verbindungen der allgemeinen Formel I sind diejenigen, in denen A 2,5-Thiophendiyl, 2,5-Furandiyl oder p-Phenylen bedeutet, $R^1$ durch die Formel II a oder II b wiedergegeben wird, $R^2$ bis $R^4$ Wasserstoff, Alkyl mit 1-6 C-Atomen, Cycloalkyl mit 5-6 C-Atomen, Methoxyäthyl oder Äthoxyäthyl bedeuten, wobei höchstens einer oder zwei der Reste $R^2$ bis $R^4$ nicht Wasserstoff bedeuten.

Gegenstand der Erfindung ist auch ein Verfahren zur Herstellung von substituierten Bisbenzimidazolylverbindungen der Formel I, das dadurch gekennzeichnet ist, daß man

a) eine Verbindung der Formel III,

$$R^6—A—R^6 \qquad (III)$$

worin A die zur Formel I gegebenen Bedeutungen hat, worin $R^6$

−CN oder 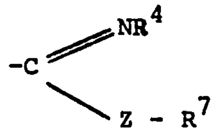

bedeutet, worin Z O oder S und $R_7$ Alkyl oder Alkoxyalkyl mit jeweils 1 bis 4 C-Atomen pro Alkylrest, insbesondere Methyl, Äthyl, n-Propyl, i-Propyl, n-Butyl oder 2-Methoxyäthyl bedeuten, mit einer Verbindung der Formel IV,

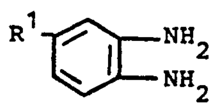

(IV)

worin $R^1$ die durch die Formel IIa oder IIb angegebene Bedeutung hat, umsetzt oder

b) eine Verbindung der allgemeinen Formel V,

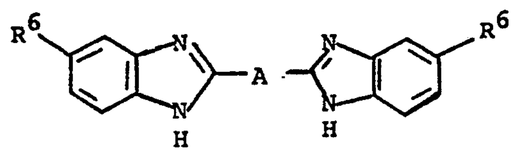

(V)

worin a die zur Formel I angegebenen Bedeutungen hat und worin $R^6$ die zur Formel III unter a) angegebenen Bedeutungen hat, mit einem Amin der Formeln VIa oder VIb,

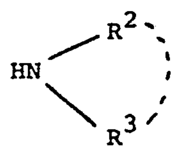
(VIa)
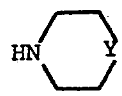
(VIb)

worin $R^2$, $R^3$ und Y die zu Formel IIa oder IIb angegebenen Bedeutungen haben, umsetzt, und gegebenenfalls eine so erhaltene Verbindung der Formel I durch Zugabe einer physiologisch verträglichen Säure HX in ihr Salz überführt, oder ein so erhaltenes Salz der Formel I durch Zugabe einer Base wei Ammoniak oder Natronlauge in eine Verbindung der Formel I überführt.

Durch Durchführung des Verfahrens a) wird eine Verbindung der Formel III mit einer Verbindung der Formel IV umgesetzt.

$a_1$) Wenn in der Formel III $R^6$ —CN bedeutet, erfolgt die Umsetzung zweckmäßig unter Zusatz von Schwefel oder schwefelhaltigen Verbindungen wie z.B. Schwefelwasserstoff, Alkali(poly)sulfiden oder Ammonium(poly)sulfiden, entweder ohne Lösungsmittel oder in einem inerten Lösungsmittel wie Benzol, Toluol, Xylol, Petroläther, Ligroin, Chloroform, Tetrachlorkohlenstoff, Dioxan oder Tetrahydrofuran. Die Reaktionstemperaturen liegen zwischen 60 und 180 °C, vorzugsweise zwischen 80 und 140 °C. Die Reaktionszeit beträgt je nach den angewendeten Reaktionstemperaturen 1-10 Stunden.

$a_2$) Wenn in der Formel III

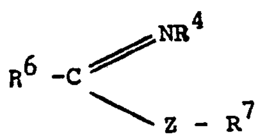

bedeutet, erfolgt die Umsetzung zweckmäßig in einem polaren Lösungsmittel in Gegenwart einer zweckmäßig physiologisch verträglichen Säure HX.

Die Reaktionstemperaturen können zwischen 0 und 180 °C, bevorzugt zwischen 20 und 120 °C liegen. Man arbeitet im allgemeinen bei der Siedetemperatur des verwendeten Lösungsmittels.

Die Reaktionszeiten liegen je nach den gegebenen Verhältnissen zwischen 30 Minuten und 5 Stunden. Als polare Lösungsmittel kommen bevorzugt Alkohole wie Methanol, Äthanol, Isopropanol, n-Propanol, n-Butanol, iso-Butanol oder Carbonsäuren wie Ameisensäure, Essigsäure, Propionsäure zur Verwendung.

Als Säure HX kommen anorganische oder organische Säuren in Betracht wie Chlor-, Brom-, Jod-Wasserstoffsäure, Phosphorsäure, Essigsäure, Propionsäure, Benzoesäure, Citronensäure, Maleinsäure, Malonsäure, Milchsäure, Weinsäure, Embonsäure, Oxalsäure, Fumarsäure, Bernsteinsäure, 2-Hydroxyäthansulfonsäure, Acetylglycin.

Nach einer bevorzugten Ausführunsform der Verfahrensvarianten $a_2$) arbeitet man mit Hydrochloriden von Imidsäureestern, wobei in der allgemeinen Formel III

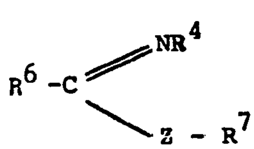

bedeutet, worin Z für Sauerstoff steht und $R^4$ und $R^7$ die zur Formel II a/b bzw. III angegebenen Bedeutungen haben. In diesem Fall sind als Lösungsmittel die unter $a_2$) angegebenen Alkohole bevorzugt.

Zur Durchführungs des Verfahrens b) wird eine Verbindung der Formel V mit einer Verbindung der Formel VIa oder VIb umgesetzt. Die Reaktionsbedingungen entsprechen insbesondere hinsichtlich der

Temperaturen, An- oder Abwesenheit von Lösungsmitteln und Reaktionszeiten den zur Durchführung des Verfahrens a) angegebenen Bedingungen, d.h. man arbeitet je nach der Bedeutung des Restes $R^6$ in der Formel V entsprechend der Verfahrensvariante $a_1$) oder $a_2$).

Die aromatischen Diamine der Formel IV können nach bekannten Verfahren (vgl. Houben-Weyl, Methoden der organischen Chemie, Band 11/1, S. 360 ff) durch Reduktion entsprechend substituierter Nitroverbindungen der Formel

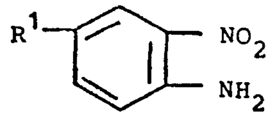

(VII)

worin $R^1$ die in den Formeln IIa-IIb angegebenen Bedeutungen hat, dargestellt werden, z.B. durch katalytische Hydrierung mit Raney-Nickel.

Die Nitroverbindungen der Formel VII werden dadurch hergestellt, daß man eine Verbindung der Formel VIII,

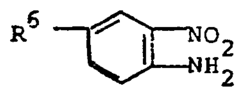

(VIII)

worin $R^6$ die zur Formel III gegebene Bedeutung hat, mit einem Amin der Formel VIa oder VIb umsetzt. die Reaktionsbedingungen entsprechen den zur Durchführung des Verfahrens a) angegebenen Bedingungen.

Die Verbindungen der allgemeinen Formel III, V und VIII werden dadurch hergestellt, daß man die jeweilige Verbindung der Formel III mit $R^6$ gleich —CN mit einem Alkohol $R^7$—OH und Chlorwasserstoff (vgl. A. Pinner. « Die Iminoäther und ihre Derivate », Berlin 1892) zum Imidsäureesterhydrochlorid umsetzt.

$R^6$ gleich 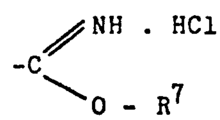

Aus den Imidsäureester-hydrochloriden werden durch Umsetzung mit Verbindungen der Formel IX

$$R^7—Z—H \qquad (IX)$$

worin $R^7$ und Z die zur Formel III genannten Bedeutungen haben, die übrigen Verbindungen der Formel III hergestellt werden, zweckmäßig bei der Siedetemperatur eines in der Verfahrensvariante $a_2$) genannt en Alkohole.

Als Amine der Formel VIa und VIb bekommen z.B. in Betracht : Ammoniak, Methylamin, Äthylamin, n-Propylamin, i-Propylamin, n-Butylamin, i-Butylamin, t-Butylamin, Pentylamin, Cyclopentylamin, Hexylamin, Cyclohexylamin, Dodecylamin, Benzylamin, Anilin, Methoxyäthylamin, Äthoxyäthylamin, N,N-Dimethylaminoäthylamin, N,N-Dimethylaminopropylamin, N,N-Diäthylaminoäthylamin, Piperidinoäthylamin, Morpholinoäthylamin, Dimethylamin, Diäthylamin, Di-n-propylamin, Di-iso-propylamin, Ditutylamin, Dicyclohexylamin, Pyrrolidin, Piperidin, Morpholin, Thiomorpholin, Piperazin, N-Methylpiperazin, N-Äthylpiperazin.

Bei der Umsetzung der aromatischen Diamine der Formel IV mit Verbindungen der allgemeinen Formel III kann der Substituent $R^1$ im Verfahrensprodukt der allgemeinen Formel I in der 5- oder 6-Position des Benzimidazols stehen. Wegen der Tautomerie des Imidazolrings und der freien Drehbarkeit zwischen Imidazol- und Brückenteil A wird jedoch jeweils nur eine einzige substituierte Bisbenzimidazolylverbindung erhalten. Die mit der Formel I beanspruchten Verbindungen umfassen deshalb alle Tautomeren und deren Gemische.

Das gleiche gilt sinngemäß für die Amidintautomerie und die Substituenten $R^2$ bis $R^4$ in den Formeln IIa bis IIb, wenn $R^2$ gleich Wasserstoff und mindestens einer der Reste $R^3$ oder $R^4$ ungleich Wasserstoff ist.

Die substituierten Bisbenzimidazolylverbindungen gemäß der Erfindung sind Chemotherapeutika und eignen sich zur Bekämpfung von parasitären Erkrankungen, insbesondere von Protozoen- und Viruserkrankungen bei Mensch und Tier.

Dabei sind sie besonders wirksam gegen Erkrankungen, die durch Trypanosomenarten, wie Trypanosoma brucei, T. rhodesiense, T. gambiense, T. evansi, T. equinum, T. equiperdum, T. congolense, T. vivax, hervorgerufen werden.

Die Verbindungen der Formel I können in Arzneimittel für Menschen und Tiere verwendet werden. Die erfindungsgemäßen Verbindungen zeichnen sich durch ein breites Wirkungsspektrum, insbesondere

auch gegen arzneimittel-resistente Stämme der obengenannten Erreger, eine niedrige Dosis curativa minima und eine gute Vertäglichkeit aus.

Die Wirkstoffe werden zusammen mit geeigneten pharmazeutischen Lösungsmitteln bzw. Trägerstoffen oral oder parenteral appliziert, wobei je nach den Umständen die eine oder andere Applikationsform bevorzugt wird. Hierfür kommen z.B. Tabletten, Kapseln, Lösungen oder Suspensionen in Betracht.

Die Dosierungen können je nach Art der Krankheitserreger in weiten Grenzen variiert werden. Eine wirksame Dosis kann so zwischen 0,1 und 300 mg pro Kilogramm Körpergewicht liegen.

Verfahren a

Herstellung der Ausgangsstoffe

Thiophen-2,5-dicarboximidsäurediäthylester-dihydrochlorid = TDD

(Formel III)

30 g Thiophen-2,5-dicarbonsäurenitril werden in 160 ml Dioxan gelöst. Nach Zugabe von 30 ml Äthanol leitet man Chlorwasserstoff-Gas bis zur Sättigung ein und hält dabei die Temperatur der Lösung unter 20 °C.

Die entstehende Suspension wird nach 24 Stunden mit Äther verrührt, abgesaugt, mit Äther gewaschen und im Exsiccator über KOH getrocknet, Fp. > 300 °C.

In analoger Verfahrensweise wird hergestellt:

Furan-2,5-dicarboximidsäurediäthylester-dihydrochlorid = FDD

(Formel III), Fp. 126-127 °C Zers., aus Furan-2,5-dicarbonsäurenitril.

Terephthalimidsäure-bis(2-methoxyäthyl)-ester-dihydrochlorid = TIB

(Formel III)

50 g Terephthalsäuredinitril werden in 500 ml Methylglykol gelöst. Man leitet HCl-Gas bis zur Sättigung ein und hält dabei die Temperatur der Lösung unter 20 °C. Die entstehende Suspension wird mit Äther verrührt, abgesaugt, mit Äther gewaschen und im Exsiccator über KOH getrocknet, Fp. > 300 °C.

In analoger Verfahrensweise wird hergestellt:

Isophthalimidsäure-bis-(2-methoxyäthyl)-ester-dihydrochlorid = IIB

(Formel III)

Fp. 141 °C Zers., aus Isophthalsäuredinitril.

4,4'-Bis-(2-methoxyäthoxycarbonimidoyl)-diphenylätherdihydrochlorid = BMD

(Formel III)

50 g 4,4'-Dicyanodiphenyläther werden in 500 ml Methylglykol suspendiert. Man leitet HCl-Gas ein, läßt die Temperatur der Lösung dabei bis auf 70 °C ansteigen und hält durch Kühlen solange bei dieser Temperatur, bis sie von selbst wieder sinkt. Man läßt über Nacht stehen, engt die Lösung im Vakuum bei maximal 45 °C ein und versetzt mit Aceton. Der Niederschlag wird abgesaugt, mit Aceton gewaschen und im Exsiccator über KOH getrocknet, Fp. 101-103 °C.

4-Amino-3-nitrobenzimidsäure-(2-methoxyäthyl)-esterhydrochlorid = ANB

(Formel VIII)

100 g 4-Amino-3-nitrobenzonitril werden in 500 ml Methylglykol suspendiert. Man leitet HCl-Gas ein, läßt die Temperatur der Lösung dabei bis auf 70 °C steigen und hält durch Kühlen solange bei dieser Temperatur, bis sie von selbst wieder sinkt. Man kühlt schließlich bis auf 0-5 °C ab, sättigt mit HCl-Gas und läßt über Nacht stehen. Anschließend wird der Niederschlag abgesaugt, mit Isopropanol gewaschen und im Exsiccator über KOH getrocknet, Fp. 220 °C.

Beispiele

Herstellung der Zwischenprodukte der Formel VII

1. 1-Amino-4-butylamidino-2-nitrobenzol-hydrochlorid

(Formel VII)

14,6 g n-Butylamin und 55,1 g 4-Amino-3-nitrobenzimidsäure-(2-methoxyäthyl)-ester-hydrochlorid (ANB) werden in 200 ml Äthanol zwei Studen unter Rückfluß gerührt. Der entstandene Niederschlag wird abgesaugt, mit Essigester gewaschen und getrocknet, Fp. 265 °C.

In analoger Verfahrensweise werden hergestellt :

Aus ANB und

2. Ammoniak :
4-Amidino-1-amino-2-nitrobenzol-hydrochlorid

3. Methylamin :
1-Amino-4-methylamidino-2-nitrobenzol-hydrochlorid

4. Äthylamin :
4-Äthylamidino-1-amino-2-nitrobenzol-hydrochlorid

5. Propylamin :
1-Amino-2-nitro-4-propylamidinobenzol-hydrochlorid

6. Isopropylamin :
1-Amino-4-isopropylamidino-2-nitrobenzol-hydrochlorid

7. Isobutylamin :
1-Amino-4-Isobutylamidino-2-nitrobenzol-hydrochlorid

8. tert. Butylamin :
1-Amino-2-nitro-4-tert. butylamidino-benzol-hydrochlorid

9. Pentylamin :
1-Amino-2-nitro-4-pentylamidinobenzol-hydrochlorid

10. Hexylamin :
1-Amino-4-hexylamidino-2-nitrobenzol-hydrochlorid

11. Cyclohexylamin :
1-Amino-4-cyclohexylamidino-2-nitrobenzol-hydrochlorid

12. Dodecylamin :
1-Amino-4-dodecylamidino-2-nitrobenzol-hydrochlorid

13. 2-Methoxyäthylamin :
1-Amino-4-(2-methoxyäthylamidino)-2-nitrobenzol-hydrochlorid

14. Benzylamin :
1-Amino-4-benzylamidino-2-nitrobenzol-hydrochlorid

15. Anilin :
1-Amino-2-nitro-4-phenylamidinobenzol-hydrochlorid

16. 2-Dimethylamino-äthylamin :
1-Amino-4-(2-dimethylamino-äthylamidino)-2-nitrobenzol-hydrochlorid

17. 3-Dimethylamino-propylamin :
1-Amino-4-(3-dimethylamino-propylamidino)-2-nitrobenzol-hydrochlorid

18. 2-Morpholino-äthylamin :
1-Amino-4-(2-morpholino-äthylamidino)-2-nitrobenzol-hydrochlorid

19. Pyrrolidin :
1-Amino-2-nitro-4-(1-pyrrolidinylcarbonimidoyl)-benzol-hydrochlorid

20. Piperidin :
1-Amino-2-nitro-4-(piperidinocarbonimidoyl)-benzol-hydroochlorid

21. Morpholin :
1-Amino-4-morpholinocarbonimidoyl-2-nitrobenzol-hydrochlorid

22. N-Methylpiperazin :
1-Amino-4-(4-methylpiperazinylcarbonimidoyl)-2-nitrobenzol-hydrochlorid

Herstellung der Zwischenprodukte der Formel IV :

23. 1,2-Diamino-4-butylamidinobenzol-hydrochlorid

(Formel IV)

44,5 g 1-Amino-4-butylamidino-2-nitrobenzol-hydrochlorid (Beispiel 1) werden in 500 ml Methanol mit einer Katalytischen Menge Raney-Nickel bei Raumtemperatur hydriert. Nach beendeter Wasserstoffaufnahme saugt man vom Katalysator ab, engt die Lösung unter vermindertem Druck etwa zur Hälfte ein und fällt das Produkt mit Essigester aus. Der Niederschlag wird abgesaugt, mit Essigester gewaschen und getrocknet, Fp. 170 °C.

In analoger Verfahrensweise werden aus den in den Biespielen 2. bis 22. aufgeführten Zwischenprodukten hergestellt :

24. aus 2. :
4-Amidino-1,2-diaminobenzol-hydrochlorid

25. aus 3. :
1,2-Diamino-4-methylaminobenzol-hydrochlorid

26. aus 4. :
4-Äthylamidino-1,2-diaminobenzol-hydrochlorid

27. aus 5. :
1,2-Diamino-4-propylamdinobenzol-hydrochlorid

28. aus 6. :
1,2-Diamino-4-isopropylamidinobenzol-hydrochlorid

29. aus 7. :
1,2-Diamino-4-isobutylamidinobenzol-hydrochlorid

30. aus 8. :
1,2-Diamino-4-tert. butylamidinobenzol-hydrochlorid

31. aus 9. :
1,2-Diamino-4-pentylamidinobenzol-hydrochlorid

32. aus 10. :
1,2-Diamino-4-hexylamidinobenzol-hydrochlorid

33. aus 11. :
1,2-Diamino-4-cyclohexylamidinobenzol-hydrochlorid

34. aus 12. :
1,2-Diamino-4-dodecylamidinobenzol-hydrochlorid

35. aus 13. :
1,2-Diamino-4-(2-methoxyäthylamidino)-benzol-hydrochlorid

36. aus 14. :
1,2-Diamino-4-benzylamidinobenzol-hydrochlorid

37. aus 15. :
1,2-Diamino-4-phenylamidinobenzol-hydrochlorid

38. aus 16. :
1,2-Diamino-4-(2-dimethylamino-äthylamidino)-benzol-hydrochlorid

39. aus 17. :
1,2-Diamino-4-(3-dimethylamino-propylamidino)-benzol-hydrochlorid

40. aus 18. :

1,2-Diamino-4-(2-morpholino-äthylamidino)-benzol-hydrochlorid

41. aus 19.:
1,2-Diamino-4-(1-pyrrolidinylcarbonimidoyl)-benzol-hydrochlorid

42. aus 20.:
1,2-Diamino-4-piperidinocarbonimidoyl-benzol-hydrochlorid

43. aus 21.:
1,2-Diamino-4-morpholinocarbonimidoyl-benzol-hydrochlorid

44. aus 22.:
1,2-Diamino-4-(4-methylpiperazinylcarbonimidoyl)-benzol-hydrochlorid

Herstellung der Endprodukte der Formel I:

45. 1,4-Bis-(5-butylamidino-2-benzimidazolyl)-benzoltetrahydrochlorid

(Formel I)
9,7 g 1,2-Diamino-4-butylamidinobenzol-hydrochlorid (Beispiel 23.) und 7,1 g TIB werden in 100 ml Methanol zwei Stunden unter Rückfluß gerührt und anschließend unter vermindertem Druck zur Trockne eingeengt.

Der Rückstand wird in Wasser heiß gelöst, filtriert und mit überschüssiger Salzsäure stark sauer gestellt.

Nach dem Erkalten wird der Niederschlag abgesaugt, mit Aceton gewaschen und getrocknet.

Das Tetrahydrochlorid hat einen Festpunkt von > 300 °C.
Zur Herstellung der freien Base wird das Hydrochlorid in Wasser gelöst und in der Siedehitze mit wässriger Ammoniaklösung versetzt. Die ausfallende freie Base schmilzt oberhalb 300 °C.

Durch Zusatz mindestens äquimolarer Mengen physiologisch verträglicher Säuren werden daraus das Acetat, Maleinat und Aceturat hergestellt.

In analoger Verfahrensweise werden aus den in den Beispielen 23. bis 44. aufgeführten Zwischenprodukten hergestellt:

46. aus 23. und TDD:
2,5-Bis-(5-butylamidino-2-benzimidazolyl)-thiophendihydrochlorid, Fp. 175 °C Zers.

47. aus 24. und TDD:
2,5-Bis-(5-amidino-2-benzimidazolyl)-thiophen-tetrahydrochlorid, Fp. > 300 °C.

48. aus 24. und FDD:
2,5-Bis-(5-amidino-2-benzimidazolyl)-furan-tetrahydrochlorid, Fp. > 300 °C

49. aus 24. und TIB:
1,4-Bis-(5-amidino-2-benzimidazolyl)-benzo-tetrahydrochlorid, Fp. > 300 °C

50. aus 24. und IIB:
1,3-Bis-(5-amidino-2-benzimidazolyl)-benzol-tetrahydrochlorid, Fp. > 300 °C

51. aus 24. und BMD:
4,4'-Bis-(5-amidino-2-benzimidazolyl)-diphenyläthertetrahydrochlorid, Fp. > 300 °C

52. aus 25. und TDD:
2,5-Bis-(5-methylamidino-2-benzimidazolyl)-thiophentetrahydrochlorid, Fp. > 300 °C

53. aus 25. und TIB:
1,4-Bis-(5-methylamidino-2-benzimidazolyl)-benzol-tetrahydrochlorid, Fp. > 300 °C.

54. aus 26. und TDD:
2,5-Bis-(5-äthylamidino-2-benzimidazolyl)-thiophendihydrochlorid, Fp. 165 °C Zers.

55. aus 26. und TIB:
1,4-Bis-(5-äthylamidino-2-benzimidazolyl)-benzoltetrahydrochlorid, Fp. > 300 °C

56. aus 27. und TDD:
2,5-Bis-(5-propylamidino-2-benzimidazolyl)-thiophentetrahydrojodid, Fp. 244 °C Zers.

57. aus 27. TIB :
1,4-Bis-(5-propylamidino-2-benzimidazolyl)-benzol-tetrahydrochlorid, Fp. > 300 °C

58. aus 28. und TDD :
2,5-Bis-(5-isopropylamidino-2-benzimidazolyl)-thiophentrihydrojodid, Fp. 203 °C Zers.

59. aus 28. und TIB :
1,4-Bis-(5-isopropylamidino-2-benzimidazolyl)-benzol-tetrahydrochlorid, Fp. > 300 °C

60. aus 29. und TDD :
2,5-Bis-(5-isobutylamidino-2-benzimidazolyl)-thiophentetrahydrochlorid, Fp. > 300 °C

61. aus 29. und TIB :
1,4-Bis-(5-isobutylamidino-2-benzimidazolyl)-benzol-tetrahydrochlorid, Fp. > 300 °C

62. aus 30. und TDD :
2,5-Bis-(5-tert. butylamidino-2-benzimidazolyl)-thiophentetrahydrochlorid, Fp. > 300 °C

63. aus 30. und TIB :
1,4-Bis-(5-tert. butylamidino-2-benzimidazolyl)-benzol-tetrahydrochlorid, Fp. > 300 °C.

64. aus 31. und TDD :
2,5-Bis-(5-pentylamidino-2-benzimidazolyl)-thiophen-tetrahydrochlorid, Fp. > 300 °C

65. aus 31. und TIB :
1,4-Bis-(5-pentylamidino-2-benzimidazolyl)-benzol-tetrahydrochlorid, Fp. > 300 °C

66. aus 32. und TDD :
2,5-Bis-(5-hexylamidino-2-benzimidazolyl)-thiophen-tetrahydrochlorid, Fp. > 300 °C

67. aus 32. und TIB :
1,4-Bis-(5-hexylamidino-2-benzimidazolyl)-benzol-tetrahydrochlorid, Fp. > 300 °C

68. aus 33. und TDD :
2,5-Bis-(5-cyclohexylamidino-2-benzimidazolyl)-thiophen-tetrahydrochlorid, Fp. > 300 °C

69. aus 33. und TIB :
1,4-Bis-(5-cyclohexylamidino-2-benzimidazolyl)-benzol-tetrahydrochlorid, Fp. > 300 °C

70. aus 34. und TDD :
2,5-Bis-(5-dodecylamidino-2-benzimidazolyl)-thiophen-tetrahydrochlorid, Fp. > 300 °C

71. aus 34. und TIB :
1,4-Bis-(5-dodecylamidino-2-benzimidazolyl)-benzol-tetrahydrochlorid, Fp. > 300 °C

72. aus 35. und TDD :
2,5-Bis-[5-(2-methoxyäthylamidino)-2-benzimidazolyl]-thiophen-tetrahydrochlorid, Fp. > 300°C

73. aus 35. und TIB :
1,4-Bis-[5-(2-methoxyäthylamidino)-2-benzimidazolyl]-benzol-tetrahydrochlorid, Fp. > 300 °C

74. aus 36. und TDD :
2,5-Bis-(5-benzylamidino-2-benzimidazolyl)-thiophen-dihydrochlorid, Fp. 210 °C Zers.

75. aus 36. und TIB :
1,4-Bis-(5-benzylamidino-2-benzimidazolyl)-benzol-tetrahydrochlorid, Fp. > 300 °C

76. aus 37. und TDD :
2,5-Bis-(5-phenylamidino-2-benzimidazolyl)-thiophen-tetrahydrochlorid, Fp. > 300 °C

77. aus 37. und TIB :
1,4-Bis-(5-phenylamidino-2-benzimidazolyl)-benzol-tetrahydrochlorid, Fp. > 300 °C

78. aus 38. und TDD :
2,5-Bis-[5-(2-dimethylamino-äthylamidino)-2-benzimidazolyl]-thiophen-tetrahydrojodid, Fp. 274-

276 °C Zers.

79. aus 39. und TIB :
1,4-Bis-[5-(3-dimethylamino-propylamidino)-2-benzimidazolyl]-benzol-tetrahydrojodid, Fp. 263-264 °C Zers.

80. aus 40. und TIB :
1,4-Bis-[5-(2-morpholino-äthylamidino)-2-benzimidazolyl]-benzol-tetrahydrojodid, Fp. 258 °C Zers.

81. aus 41. und TDD :
2,5-Bis-[5-(1-pyrrolidinylcarbonimidoyl)-2-benzimidazolyl]-thiophen-dihydrojodid, Fp. > 300 °C

82. aus 41. und FDD :
2,5-Bis-[5-(1-pyrrolidinylcarbonimidoyl)-2-benzimidazolyl]-furan-tetrahydrojodid, Fp. > 300 °C

83. aus 41. und TIB :
1,4-Bis-[5-(1-pyrrolidinylcarbonimidoyl)-2-benzimidazolyl]-benzol-tetrahydrochlorid, Fp. > 300 °C

84. aus 41. und BMD :
4,4'-Bis-[5-(1-pyrrolidinylcarbonimidoyl)-2-benzimidazolyl]-diphenyläther-diacetat, Fp. 151 °C Zers.

85. aus 42. und TDD :
2,5-Bis-(5-piperidinocarbonimidoyl-2-benzimidazolyl)-thiophen-dihydrojodid, Fp. 260 °C Zers.

86. aus 43. und TDD :
2,5-Bis-(5-morpholinocarbonimidoyl-2-benzimidazolyl)-thiophen-dihydrojodid, Fp. 260 °C Zers.

87. aus 43. und FDD :
2,5-Bis-(5-morpholinocarbonimidoyl-2-benzimidazolyl)-furan-dihydrojodid, Fp. 270 °C Zers.

88. aus 43. und TIB :
1,4-Bis-(5-morpholinocarbonimidoyl-2-benzimidazolyl)-benzol-dihydrochlorid, Fp. 283 °C Zers.

89. aus 43. aus BMD :
4,4'-Bis-(5-morpholinocarbonimidoyl-2-benzimidazolyl)-diphenyläther-tetraacetat, Fp. 158 °C Zers.

90. aus 44. und TDD :
2,5-Bis-[5-(4-methylpiperazinylcarbonimidoyl)-2-benzimidazolyl]-thiophen-dihydrojodid, Fp. 233 °C Zers.

## Verfahren b

### Herstellung der Ausgangsstoffe

2,5-Bis-(5-cyano-2-benzimidazolyl)-thiophen

(Formel V)
30 g Thiophen-2,5-dicarboximidsäurediäthylester-dihydrochlorid werden in 250 ml Methanol mit 26,7 g 3,4-Diaminobenzonitril 4 Stunden unter Rückfluß gekocht. Die entstehende Fällung wird abgesaugt, mit Wasser gewaschen und aus 800 ml Methylglykol umkristallisiert, Fp. > 300 °C.

2,5-Bis-[5-(2-methoxyäthoxycarbonimidoy)-2-benzimidazolyl]-thiophen-tetrahydrochlorid = BBT

(Formel V)
15,5 g 2,5-Bis-(5-cyano-2-benzimidazolyl)-thiophen werden in 155 ml Methylglykol suspendiert. Man leitet HCl-Gas bis zur Sättigung ein und hält dabei die Temperatur der Lösung unter 20 °C.
Die entstehende Suspension wird nach 24 Stunden mit Äther verrührt, abgesaugt, mit Äther gewaschen und im Exsiccator über KOH getrocknet, Fp. 273 °C (Zers.).
In analoger Verfahrensweise wird hergestellt :

2,5-Bis-[5-(2-methoxyäthoxycarbonimidoyl)-2-benzimidazolyl]-furan-tetrahydrochlorid = BBF
1,4-Bis-[5-(2-methoxyäthoxycarbonimidoyl)-2-benzimidazolyl]-benzol-tetrahydrochlorid = PBB
1,3-Bis-[5-(2-methoxyäthoxycarbonimidoyl)-2-benzimidazolyl]-benzol-tetrahydrochlorid = MBB
4,4'-Bis-[5-(2-methoxyäthoxycarbonimidoyl)-2-benzimidazolyl]-diphenyläther-tetrahydrochlorid = BBD

Durch Umsetzung dieser Imidsäureester mit entsprechenden Aminen werden die gleichen Endprodukte der Formel I wie in den Beispielen 45. bis 90. des Verfahrens a erhalten.

Beispiele

91. 2,5-Bis-(5-amidino-2-benzimidazolyl)-thiophentetrahydrochlorid

(Formel I)

6,6 g 2,5-Bis-5-(2-methoxyäthoxycarbonimidoyl)-2-benzimidazolyl-thiophen-tetrahydrochlorid (BBT) werden in 100 ml Methanol suspendiert. Unter Rühren leitet man fünf Stunden lang trockenen Ammoniak ein und bringt anschließend unter vermindertem Druck zur Trockne. Der Rückstand wird in Wasser Heiß gelöst, filtriert und mit überschüssiger Salzsäure stark sauer gestellt. Nach dem Erkalten wird der Niederschlag abgesaugt, mit Aceton gewaschen und getrocknet, Fp. > 300 °C

In analoger Verfahrensweise werden z.B. hergestellt ;

92. aus Ammoniak und BBF :
2,5-Bis-(5-amidino-2-benzimidazolyl)-furantetrahydrochlorid, Fp. > 300 °C

93. aus Ammoniak und PBB :
1,4-Bis-(5-amidino-2-benzimidazolyl)-benzoltetrahydrochlorid, Fp. > 300 °C

94. aus Ammoniak und MBB :
1,3-Bis-(5-amidino-2-benzimidazolyl)-benzoltetrahydrochlorid, Fp. > 300 °C

95. aus Ammoniak und BBD :
4,4'-Bis-(5-amidino-2-benzimidazolyl)-diphenyläther-tetrahydrochlorid, Fp. > 300 °C

**Ansprüche** (für die Vertragsstaaten : BE, CH, DE, FR, GB, IT, NL)

1. Substituierte Bisbenzimidazolylverbindungen der allgemeinen Formel I

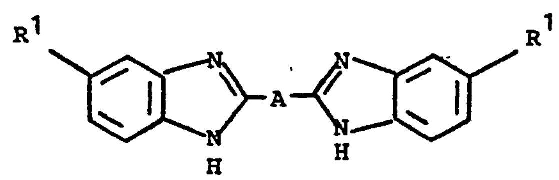

(I)

und deren physiologisch verträgliche Salze, worin A 2,5-Thiophendiyl, 2,5-Furandiyl, p-Phenylen, m-Phenylen oder 4,4'-Phenoxyphenylen bedeutet und worin $R^1$ durch eine der allgemeinen Formel IIa oder IIb wiedergegeben wird,

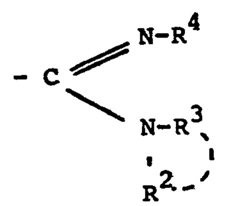

(IIa)

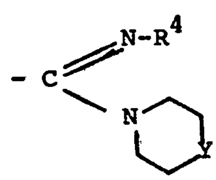

(IIb)

worin die Reste $R^2$ bis $R^4$ unabhängig voneinander Wasserstoff, Alkyl mit1-18, Cycloalkyl mit 5-8 C-Atomen, Aminoalkyl, N-Alkylaminoalkyl, N,N-Dialkylaminoalkyl, wobei eine Alkylgruppe jeweils 1-4 C-Atome aufweist, Morpholinoäthyl, Methoxyäthyl, Äthoxyäthyl, Benzyl, Phenäthyl oder Phenyl bedeuten, oder worin die Rest $R^2$ und $R^3$ zusammen eine Alkylenbrücke mit 4, 5 oder 6 $CH_2$-Gruppen bedeuten, und worin Y O, S, NH oder N-$R^5$ bedeutet, wobei $R^5$ für Alkyl mit 1-4 C-Atomen steht.

2. Verbindungen der Formel 1 in Anspruch 1, worin A 2,5-Thiophendiyl, 2,5-Furandiyl oder p-Phenylen bedeutet, $R^1$ durch die Formel II a oder II b wiedergegeben wird, $R^2$ bis $R^4$ Wasserstoff, Alkyl mit 1-6 C-Atomen, Cycloalkyl mit 5-6 C-Atomen, Methoxyäthyl oder Äthoxyäthyl bedeuten, wobei höchstens einer oder zwei der Reste $R^2$ bis $R^4$ nicht Wasserstoff bedeuten, und die Salze dieser Verbindungen mit physiologisch verträglichen Säuren.

3. Verfahren zur Herstellung von substituierten Bis-benzimidazolylverbindungen der Formel I in Anspruch 1, dadurch gekennzeichnet, daß man

a) eine Verbindung der Formel III

$R^6—A—R^6$ (III)

worin A die in Anspruch 1 angegebenen Bedeutungen hat, und worin $R^6$

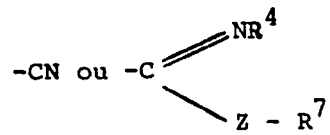

bedeutet, worin Z O oder S und $R^7$ Alkyl oder Alkoxyalkyl mit jeweils 1 bis 4 C-Atomen pro Alkylrest bedeuten, mit einer Verbindung der Formel IV,

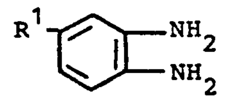

(IV)

worin $R^1$ die in den Formeln IIa oder IIb angegebenen Bedeutungen hat, umsetzt oder

b) eine Verbindung der allgemeinen Formel V,

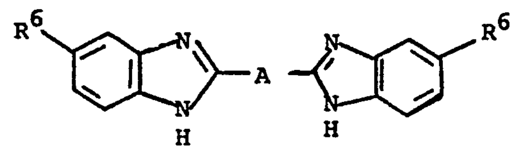

(V)

worin A die in Anspruch 1 angegebenen Bedeutungen hat, und worin $R^6$ die zur Formel III unter a) angegebenen Bedeutungen hat, mit einem Amin der Formel VIa oder VIb,

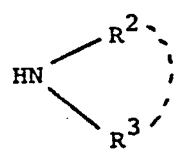

(VIa)

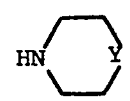

(VIb)

worin $R^2$, $R^3$ und Y die zu den Formeln IIa oder IIb angegebenen Bedeutungen haben, umsetzt, und gegebenenfalls eine so erhaltene Verbindung der Formel I durch Zugabe einer physiologisch verträglichen Säure HX in ihr Salz überführt, oder ein so erhaltenes Salz der Formel I durch Zugabe einer Base in eine Verbindung der Formel I überführt.

4. Arzneimittel, gekennzeichnet durch einen Gehalt einer Verbindung der Formel I in Anspruch 1, in Mischung mit einem pharmazeutisch üblichen Trägerstoff und/oder Konstituens.

**Anspruch** (für den Vertragsstaat AT)

Verfahren zur Herstellung von substituierten Bis-benzimidazolylverbindungen der Formel I,

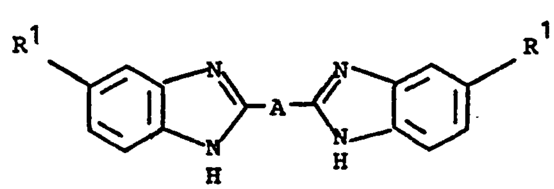

(I)

und deren physiologisch verträgliche Salze, worin A 2,5-Thiophendiyl, 2,5-Furandiyl, p-Phenylen, m-Phenylen oder 4,4'-Phenoxyphenylen bedeutet und worin $R^1$ durch eine der allgemeinen Formel IIa oder 11b wiedergegeben wird,

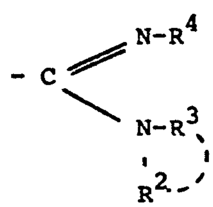

(IIa)

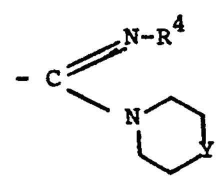

(IIb)

worin die Reste $R^2$ bis $R^4$ unabhängig voneinander Wasserstoff, Alkyl mit 1-18, Cycloalkyl mit 5-8 C-Atomen, Aminoalkyl, N-Alkylaminoalkyl, N,N-Dialkylaminoalkyl wobei eine Alkylgruppe jeweils 1-4 C-Atome aufweist, Morpholinoäthyl, Methoxyäthyl, Äthoxyäthyl, Benzyl, Phenäthyl oder Phenyl bedeuten, oder worin die Reste $R^2$ und $R^3$ zusammen eine Alkylenbrücke mit 4, 5 oder 6 $CH_2$-Gruppen bedeuten, und worin Y O, S, NH oder N-$R^5$ bedeutet, wobei $R^5$ für Alkyl mit 1-4 C-Atomen steht, dadurch gekennzeichnet, daß man

a) eine Verbindung der Formel III

$$R^6—A—R^6 \qquad (III)$$

worin A die in Anspruch 1 angegebenen Bedeutungen hat, und worin $R^6$

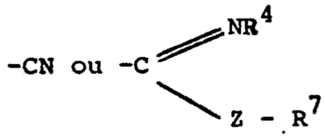

bedeutet, worin Z O oder S und $R^7$ Alkyl oder Alkoxyalkyl mit jeweils 1 bis 4 C-Atomen pro Alkylrest bedeuten, mit einer Verbindung der Formel IV,

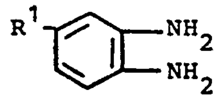

(IV)

worin $R^1$ die in den Formeln IIa oder IIb angegebenen Bedeutungen hat, umsetzt oder

b) eine Verbindung der allgemeinen Formel V,

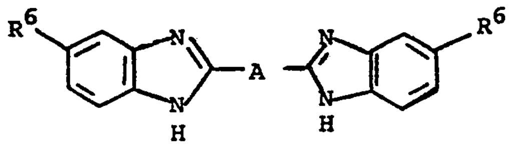

(V)

worin A die in Anspruch 1 angegebenen Bedeutungen hat, und worin $R^6$ die zur Formel III unter a) angegebenen Bedeutungen hat, mit einem Amin der Formel VIa oder VIb,

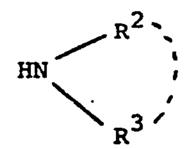

(VIa)

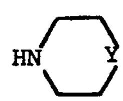

(VIb)

worin $R^2$, $R^3$ und Y die zu den Formeln IIa oder IIb angegebenen Bedeutungen haben, umsetzt, und gegebenenfalls eine so erhaltene Verbindung der Formel I durch Zugabe einer physiologisch verträglichen Säure HX in ihr Salz überführt, oder ein so erhaltenes Salz der Formel I durch Zugabe einer Base in eine Verbindung der Formel I überführt.

**Claims** (for contracting States : BE, CH, DE, FR, GB, IT, NL)

1. Substituted bis-benzimidazolyl compounds of the general formula I

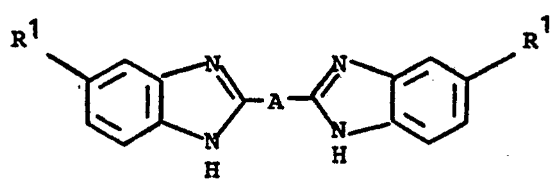

(I)

and their physiologically compatible salts, in which A denotes 2,5-thiophenediyl, 2,5-furanediyl, p-phenylene, m-phenylene or 4,4'-phenoxyphenylene, and in which $R^1$ is represented by one of the general formulae IIa or IIb

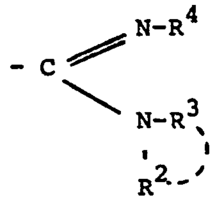 (IIa) 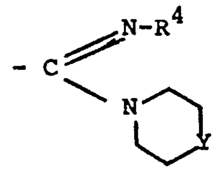 (IIb)

in which the radicals $R^2$ to $R^4$ independently of one another denote hydrogen, alkyl with 1-18 C atoms, cycloalkyl with 5-8 atoms, aminoalkyl, N-alkylaminoalkyl or N,N-dialkylaminoalkyl, in which an alkyl group in each case has 1-4 C atoms, morpholinoethyl, methoxyethyl, ethoxyethyl, benzyl, phenethyl or phenyl, or in which the radicals $R^2$ and $R^3$ together denote an alkylene bridge with 4, 5 or 6 $CH_2$ groups, and in which Y denotes O, S, NH or N-$R^5$, in which $R^5$ represents alkyl with 1-4 C atoms.

2. Compounds of formula I in claim 1, in which A denotes 2,5-thiophenediyl, 2,5-furandiyl or p-phenylene, $R^1$ is represented by formula IIa or IIb, $R^2$ to $R^4$ denote hydrogen, alkyl with 1-6 C atoms, cycloalkyl with 5-6 C-atoms, methoxyethyl or ethoxyethyl, wherein only one or two of the radicals $R^2$ to $R^4$ are different from hydrogen, and the salts of these compounds with physiologically compatible acids.

3. Process for the manufacture of substituted bis-benzimidazolyl compounds of the formula I in claim 1, which comprises

a) reacting a compound of the formula III

$$R^6—A—R^6 \qquad (III)$$

in which A has the meanings indicated in claim 1 and in which $R^6$ denotes -CN or

-CN ou 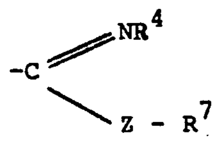

in which Z denotes O or S and $R^7$ denotes alkyl or alkoxyalkyl with, in each case, 1 to 4 C atoms per alkyl radical, with a compound of the formula IV

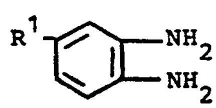 (IV)

in which $R^1$ has the meanings indicated by the formula IIa or IIb, or

b) reacting a compound of the general formula V

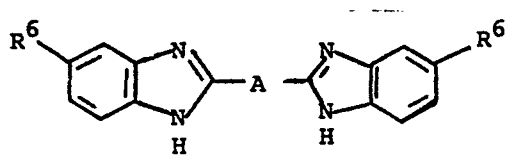 (V)

in which A has the meanings indicated in claim 1 and in which $R^6$ has the meanings indicated under a) under formula III, with an amine of the formula VIa or VIb

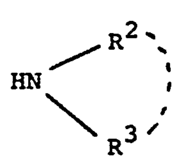 (VIa) 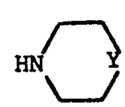 (VIb)

in which $R^2$, $R^3$ and Y have the meanings indicated under formula IIa or IIb, and optionally converting a compound of the formula I, which is thus obtained, into its salt by adding a physiologically compatible acid HX, or converting a salt of the formule I, which is thus obtained, into a compound of the formula I by adding a base.

4. Pharmaceutical composition containing a compound of the formula I in claim 1, in admixture with a pharmaceutically conventional excipient and/or constituent.

**Claim** (for Contracting State AT)

Process for the manufacture of substituted bis-benzimidazolyl compounds of the general formula I

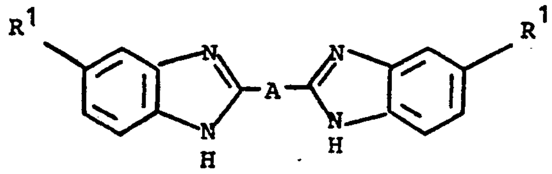

(I)

and their physiologically compatible salts, in which A denotes 2,5-thiophenediyl, 2,5-furanediyl, p-phenylene, m-phenylene or 4,4'-phenoxyphenylene, and in which $R^1$ is represented by one of the general formulae

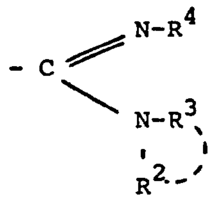 (IIa) 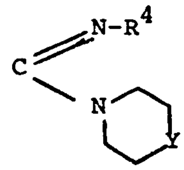 (IIb)

in which the radicals $R^2$ to $R^4$ independently of one another denote hydrogen, alkyl with 1-18 C atoms, cycloalkyl with 5-8 atoms, aminoalkyl, N-alkylaminoalkyl or N,N-dialkylaminoalkyl, in which an alkyl group in each case has 1-4 C atoms, morpholinoethyl, methoxyethyl, ethoxyethyl, benzyl, phenethyl or phenyl, or in which the radicals $R^2$ and $R^3$ together denote an alkylene bridge with 4, 5 or 6 $CH_2$ groups, and in which Y denotes O, S, NH or N-$R^5$, in which $R^5$ represents alkyl with 1-4 C atoms, which comprises

a) reacting a compound of the formula III

$$R^6—A—R^6 \qquad \text{(III)}$$

in which A has the meanings indicated above and in which $R^6$ denotes

-CN ou 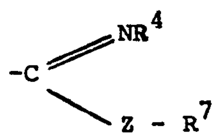

in which Z denotes O or S and $R^7$ denotes alkyl or alkoxyalkyl with, in each case, 1 to 4 C atoms per alkyl radical, with a compound of the formula IV

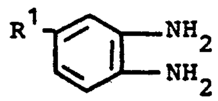

(IV)

in which $R^1$ has the meanings indicated by the formula IIa or IIb, or

b) reacting a compound of the general formula V

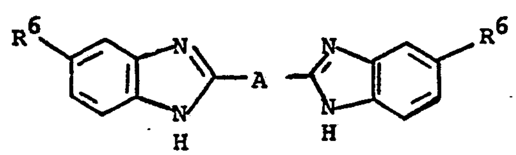

(V)

in which A has the meanings indicated above and in which $R^6$ has the meanings indicated under a) under formula III, with an amine of the formula VIa or VIb

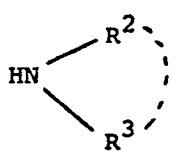 (VIa) 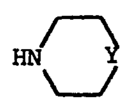 (VIb)

in which $R^2$, $R^3$ and Y have the meanings indicated under formula IIa or IIb, and optionally converting a compound of the formula I, which is thus obtained, into its salt by adding a physiologically compatible acid HX, or converting a salt of the formula I, which is thus obtained, into a compound of the formula I by adding a base.

**Revendications** (pour les Etats contractants : BE, CH, DE, FR, GB, IT, NL)

1. Composés de bisbenzimidazolyle substitués répondant à la formule générale I

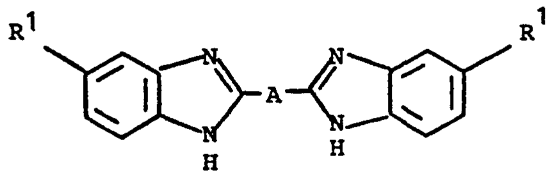

(I)

dans laquelle A représente un radical 2,5-thiophenediyle, 2,5-furannediyle, p-phénylène, m-phénylène ou 4,4'-phénoxyphénylène et $R^1$ répond à une des formules générales IIa ou IIb

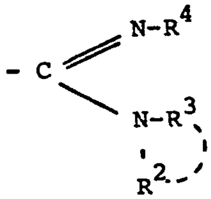

(IIa)

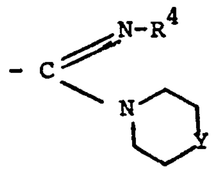

(IIb)

où les radicaux $R^2$ à $R^4$ représentent indépendamment l'un de l'autre un atome d'hydrogène ou un radical alkyle ayant de 1 à 18 atomes de carbone, cycloalkyle ayant de 5 à 8 atomes de carbone, aminoalkyle, N-alkylaminoalkyle, N,N-dialkylaminoalkyle, où un groupe alkyle comprend chaque fois 1 à 4 atomes de carbone, morpholinoéthyle, méthoxyéthyle, éthoxyéthyle, benzyle, phényléthyle, ou phényle, où les radicaux $R^2$ et $R^3$ pouvant former conjointement un pont alkylène comprenant 4, 5 ou 6 groupes $CH_2$, et Y représente un atome d'oxygène ou de soufre ou un groupe NH ou N-$R^5$, où $R^5$ représente un radical alkyle ayant de 1 à 4 atomes de carbone, et les sels physiologiquement compatibles de ces composés.

2. Composés de formule I suivant la revendication 1, caractérisés en ce que A représente un radical 2,5-thiophènediyle, 2,5-furannediyle ou p-phénylène, $R^1$ répond à l'une des formules IIa ou IIb où $R^2$ a $R^4$ représentent un atome d'hydrogène ou un radical alkyle ayant de 1 à 6 atomes de carbone, cycloalkyle ayant de 5 à 6 atomes de carbone, méthoxyéthyle ou éthoxyéthyle au maximum 1 ou 2 des radicaux $R^2$ à $R^4$ ne représentant pas d'hydrogène, et les sels de ces composés avec des acides physiologiquement compatibles.

3. Procédé de préparation de composés de bisbenzimidazolyle substitués de la formule I, selon la revendication 1, caractérisé en ce que

a) on fait réagir un composé de la formule III

$$R^6—A—R^6 \quad (III)$$

dans laquelle A a la même signification que celle donnée dans la revendication 1 et $R^6$ représente un groupe

$-CN$ ou 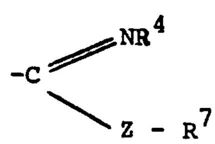

où Z représente un atome d'oxygène ou de soufre, et $R^7$ représente un radical alkyle ou alcoxyalkyle ayant respectivement 1 à 4 atomes de carbone par groupe alkyle, avec un composé de la formule IV

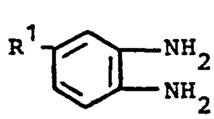

(IV)

dans laquelle $R^1$ a la signification donnée pour les formules IIa ou IIb, ou

b) on fait réagir un composé de la formule générale V

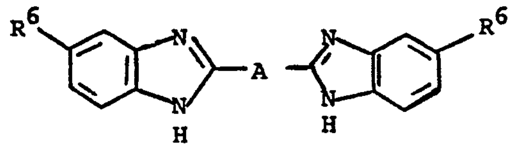 (V)

dans laquelle A a la même signification que celle donnée dans la revendication 1 et $R^6$ a la même signification que celle donnée pour la formula III sous a), avec une amine de la formula VIa ou VIb

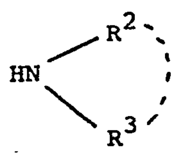 (VIa) 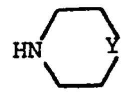 (VIb)

dans lesquelles $R^2$, $R^3$ et Y ont la même signification que celle donnée pour les formules IIa et IIb et éventuellement on convertit un composé de la formule I ainsi obtenu en son sel par addition d'un acide HX physiologiquement compatible, ou on convertit un sel de la formule I ainsi obtenu en un composé de la formule I par addition d'une base.

4. Médicament, caractérisé par une teneur en un composé de la formule I suivant la revendication 1, en mélange avec un support et/ou un adjuvant pharmaceutiquement courant.

**Revendication** (pour l'Etat contractant AT)

Procédé de préparation de composés de bisbenzimidazolyle substitués répondant à la formule I,

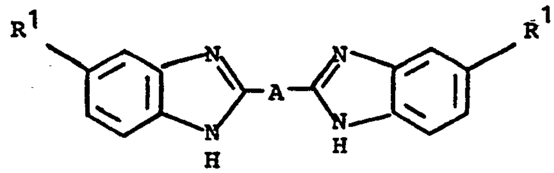 (I)

dans laquelle A représente un radical 2,5-thiophènediyle, 2,5-furanediyle, p-phénylène, m-phénylène ou 4,4'-phénoxyphénylène et $R^1$ répond à une des formules générales IIa ou IIb

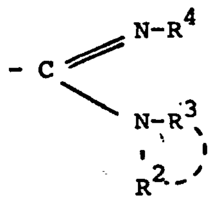 (IIa) 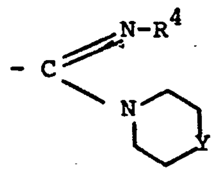 (IIb)

où les radicaux $R^2$ à $R^4$ représentent indépendamment l'un de l'autre un atome d'hydrogène ou un radical alkyle ayant de 1 à 18 atomes de carbone, cycloalkyle ayant de 5 à 8 atomes de carbone, aminoalkyle, N-alkylaminoalkyle, N,N-dialkylaminoalkyle, où un groupe alkyle comprend chaque fois 1 à 4 atomes de carbone, morpholinoéthyle, méthoxyéthyle, éthoxyéthyle, benzyle, phényléthyle, ou phényle, ou les radicaux $R^2$ et $R^3$ pouvant former conjointement un pont alkylène comprenant 4, 5 ou 6 groupes $CH_2$, et Y représente un atome d'oxygène ou de soufre ou un groupe NH ou N-$R^5$, où $R^5$ représente un radical alkyle ayant de 1 à 4 atomes de carbone, et les sels physiologiquement compatibles de ces composés, caractérisé en ce que

a) on fait réagir un composé de la formule III

$$R^6—A—R^6 \qquad (III)$$

dans laquelle A a la même signification que celle donnée pour la formule I et $R^6$ représente un groupe

-CN ou 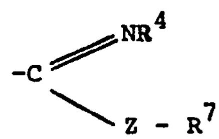

où Z représente un atome d'oxygène ou de soufre, et $R^7$ représente un radical alkyle ou alcoxyalkyle ayant respectivement 1 à 4 atomes de carbone par groupe alkyle, avec un composé de la formule IV

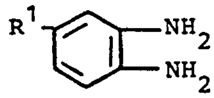

(IV)

dans laquelle $R^1$ a la signification donnée pour les formules IIa ou IIb, ou

b) on fait réagir un composé de la formule générale V

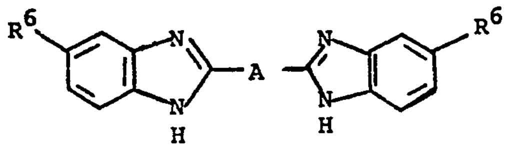

(V)

dans laquelle A a la même signification que celle donnée pour la formule I et $R^6$ a la même signification que celle donnée pour la formule III sous a avec une amine de la formule VIa ou VIb

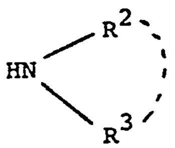

(VIa)

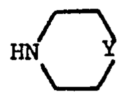

(VIb)

dans lesquelles $R^2$, $R^3$ et Y ont la même signification que celle donnée pour les formules IIa et IIb et éventuellement on convertit un composé de la formule I ainsi obtenu en son sel par addition d'un acide HX physiologiquement compatible, ou on convertit un sel de la formule I ainsi obtenu en un composé de la formule I par addition d'une base.